# EUROPEAN PATENT SPECIFICATION

(11) **EP 3 403 662 B1**
(45) Date of publication and mention of the grant of the patent: **29.01.2020**
(21) Application number: 18168608.0
(22) Date of filing: 20.04.2018
(51) Int. Cl.: A61K 36/05, A23L 33/10

(54) **PHARMACOACTIVE NUTRIENT AND A PROCESS OF PRODUCTION THEREOF FROM MARINE ALGAE**
PHARMAKOAKTIVER NÄHRSTOFF UND VERFAHREN ZUR HERSTELLUNG DAVON AUS MEERESALGEN
NUTRIMENT PHARMACOACTIF ET SON PROCÉDÉ DE PRODUCTION À PARTIR D'ALGUES MARINES

(30) Priority: 02.05.2017 IN 201741015524
(43) Date of publication of application: 21.11.2018
(73) Proprietor: National Institute of Ocean Technology, 600100 Chennai, Tamil Nadu (IN)
(72) Inventor: RAMALINGAM, Kirubagaran, 600100 Chennai (IN); ANTHONY, Josephine, 600100 Chennai (IN); THALAVAI SHIVASANKARASUBBIAH, Kumar, 600100 Chennai (IN); RANGAMARAN, Vijaya Raghavan, 600100 Chennai (IN); GOPAL, Dharani, 600100 Chennai (IN); NAMBALI VALSALAN, VinithKumar, 600100 Chennai (IN); JOSEPH, Mary, Leema Thilakam, 600100 Chennai (IN); DHASSIAH, Magesh, Peter, 600100 Chennai (IN)
(74) Representative: HGF Limited

(56) References cited:
- EP-A1- 3 246 311
- WO-A1-2006/095964
- WO-A1-2012/089841
- CN-A- 106 501 191
- JP-A- H0 975 094
- US-A1- 2011 207 952

## Description

### FIELD

The present disclosure relates to the field of marine algal pharmacoactive nutrient and a process of production thereof.

### BACKGROUND

*Chlorella* is a unicellular green algae which occurs in both fresh water and marine water. It is also known as a sun-powered supernutrient because of its beneficial properties which include wound healing, detoxification, constipation relief and growth stimulation. Because of such beneficial properties *chlorella* biomass is widely employed as a supplementary food.

*Chlorella* has the highest content of chlorophyll than any known plant. It also contains vitamins, minerals, dietary fiber, nucleic acids, amino acids, enzymes, etc. The vitamin content consists of provitaminA, vitamins B1, B2, B6, niacin, B12, biotin, vitamin C, vitamin K, pantothenic acid, folic acid, choline, lipoic acid, ionositol, 4-Aminobenzoic acid (PABA) etc. Chlorella Growth Factor (CGF) derived from fresh water microalgae, using hot water treatment, is a nucleotide-peptide complex, rich in carbohydrates, vitamins and minerals, and commercially available as a nutritional supplement in the form of tablets and syrups.

Generally, the commercial products of CGF, are prepared using hot water treatment, exclusively from the dry biomass of microalgae which are cultured in freshwater. The dry biomass used in the preparation of the CGF, the dry (dead) biomass expels out only the natural and inherent contents. In contrast, when heat treatment is carried out with the wet (live) biomass, this treatment induces those substances to get transformed into certain novel heat shock proteins to combat the thermal stress, which are of high biological importance. Further, the use of live biomass circumvents the intense energy involved in drying, reduces the loss of by-products as a result of oxidation during drying and also curtails the possible fungal contamination during drying.

Moreover, for the cultivation of freshwater algae, large quantity of potable water and vast area of fertile cultivable farmland are utilized. Further marine microalgae are impedingly prolific sources of valuable bioactive compounds that signify the development of new pharmaceutical strategies. There is, therefore, felt a need for producing an extract from marine microalgae using seawater on uncultivable coastal land. WO 2006/095964A1 relates to a method of extracting a liquid extract directly from a crude culture medium having chlorella grown therein, by heating the crude culture medium so as to extract a content in which nutrients are contained in the chlorella cell membrane; separating the content extracted in the heating/extraction step into an insoluble cell membrane and a beneficial substance by means of a centrifuge; filtering the beneficial substance separated in the cell separation step; and sterilizing the beneficial substance resulting from the filtration step.

### OBJECTS

Some of the objects of the present disclosure, which at least one embodiment herein satisfies, are as follows:
It is an object of the present disclosure to ameliorate one or more problems of the prior art or to at least provide a useful alternative.

Another object of the present disclosure is to provide a pharmacoactive nutrient.

Still another object of the present disclosure is to provide a pharmacoactive nutrient from a live marine algae.

Yet another object of the present disclosure is to provide a process for production of the pharmacoactive nutrient from live marine algae.

Still another object of the present disclosure is to provide an algal culture medium comprising the pharmacoactive nutrient for enhancing algal biomass.

Other objects and advantages of the present disclosure will be more apparent from the following description.

### SUMMARY

The present disclosure envisages a pharmacoactive nutrient medium and a process for preparation of the same. In the process of the present disclosure, initially predetermined ratio of water and live marine algae are added in a reaction vessel followed by through mixing to form aqueous slurry. This aqueous slurry is then heated to a temperature in the range of 90 to 110 °C for a time period in the range of 25 to 35 minutes to form a first mixture. The first mixture is then cooled till it attains a temperature of 25 to 30 °C to form a cooled mixture. This cooled mixture is then subjected to centrifugation at a speed of 6000 to 10000 rpm in an environment maintained at 4 °C until the cooled mixture separates into a supernatant and a residue. The supernatant is then separated from the residue and concentrated to 1/4^{th} of its total volume using concentrator at 4°Cto obtain a concentrated pharmacoactive nutrient medium.

In an embodiment of the present disclosure, the first mixture is maintained at a temperature in the range of 90 to 110 °C for 25 to 35 minutes. In another embodiment of the present disclosure, the live marine algae is first washed with sterile water at least once and then added in the reaction vessel. Typically, the centrifugation is carried out for 20 to 40 minutes at 4 °C. Typically, the live marine alga is *Chlorella vuglaris.* In still another embodiment of the present disclosure, the separation of the supernatant is carried out by at least one method selected from the group consisting of, but not limited to, decantation and filtration. In yet another embodiment of the present disclosure, the pharmacoactive nutrient medium is substantially devoid of heavy metals. Typically, the ratio of water and live marine algae is in the range of 100:10 to100:15.

### BRIEF DESCRIPTION OF THE ACCOMPANYING DRAWING

The present disclosure will now be described with the help of the accompanying drawing, in which:
**Figure 1** illustrates macromolecular content of the hot water extracts (HWEs) of marine microalgae, *Chlorella vuglaris;*
   wherein 1 denotes W-HWE I; 2 denotes D-HWE I; 3 denotes W-HWE II; and 4 denotes D-HWE II;
**Figure 2** illustrates energy value of different HWEs of marine microalgae, *Chlorella vuglaris;* wherein 1 denotes W-HWE I; 2 denotes D-HWE I; 3 denotes W-HWE II; and 4 denotes D-HWE II;
**Figure 3a** illustrates the MALDI-MS2 spectrum of the W-HWE I prepared from the wet biomass of marine microalgae, *Chlorella vuglaris;*
**Figure 3b** illustrates the MALDI-MS2 spectrum of the D-HWE I prepared from the dry biomass of marine microalgae, *Chlorella vuglaris;*
**Figure 3c** illustrates the MALDI-MS2 spectrum of W-HWE II prepared from the dry biomass of marine microalgae, *Chlorella vuglaris;* and
**Figure 3d** illustrates the MALDI-MS2 spectrum of D-HWE II prepared from the dry biomass of marine microalgae, *Chlorella vuglaris;*
**Figure 4a** illustrates the biomass production in f/2 medium containing W-HWE II; wherein 1 denotes control; and 2 denotes W-HWE II; and
**Figure 4b** illustrates the determination of cell count in f/2 medium containing W-HWE II; wherein 1 denotes control; and 2 denotes W-HWE II;
**Figure 5a** illustrates the sleeping test for assessing central nervous system depressant action;
   wherein 1 denotes on set sleeping; 2 denotes back of time; 3 denotes total duration;
   A denotes PB (40 mg/kg); B denotes PB (40 mg/kg) + CP (5 mg/kg); C denotes PB (40 mg/kg) +W-HWE II (25 mg/kg); and D denotes PB (40 mg/kg) + W-HWE II (50 mg/kg) and the values are mean of six independent experiments; and

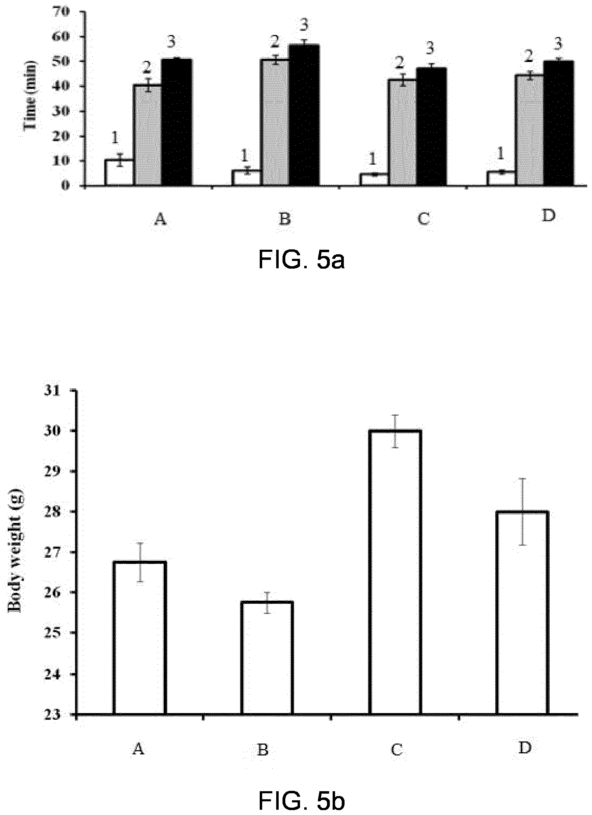

**Figure 5b** illustrates the body weight of mice during PB- induced central nervous system depression;
   wherein A denotes PB (40 mg/kg); B denotes PB (40 mg/kg) + CP (5 mg/kg); C denotes PB (40 mg/kg) + W-HWE II (25 mg/kg); and D denotes PB (40 mg/kg) + W-HWE II (50 mg/kg) and the values are mean of six independent experiments.

### DETAILED DESCRIPTION

*Chlorella* is a genus of single-cell green algae belonging to the phylum Chlorophyta. It is spherical in shape, about 2 to 10 µm in diameter, and is without flagella. *Chlorella* contains the green photosynthetic pigments chlorophyll-a and -b in its chloroplast. Through photosynthesis, it multiplies rapidly, requiring only carbon dioxide, water, sunlight, and a small amount of minerals to reproduce. Moreover, it is also known as a sun-powered supernutrient because of its beneficial properties which include wound healing, detoxification, constipation relief and growth stimulation. Because of such beneficial properties *chlorella* biomass is widely employed as a supplementary food.

The present disclosure envisages a pharmacoactive nutrient medium and a process for preparation of the same. The present disclosure also discloses a methodology for efficient extraction of nutritionally and pharmaceutically valuable extract from the live biomass of marine microalgae. Typically, the live marine algae is selected from the group consisting of *Chlorella vuglaris, Chlorella pyrenoidosa, Chlorella minutissima* and *Chlorella sorokiniana.*

In an embodiment of the present disclosure, disclosed is a process of producing the Pharmacoactive nutrient from the live biomass of marine microalga strain, *Chlorella vuglaris.*

In an embodiment of the present disclosure, a fast growing, high biomass and lipid producing microalgal strain, *Chlorella vulgaris* isolated from the Pondicherry coast (11° 54' 27.54" N; 79° 49' 35.70" E) was cultured in natural filtered seawater with f/2 medium. Mutant strains of *Chlorella vulgaris,* either naturally occurring or artificially produced, for example by irradiation (e.g. ultraviolet, X-ray), chemical mutagens or by site-directed mutagenesis, are within the scope of the present disclosure.

In the process of the present disclosure, initially predetermined ratio of water and live marine algae are added in a reaction vessel followed by thorough mixing to form an aqueous slurry. In an embodiment of the present disclosure, the alga is a marine algae. Typically, the water is sterile MilliQ water obtained from Milli-Q water purifiers created by Millipore Corporation. The so obtained aqueous slurry is then subjected to a heat treatment at a predetermined temperature for a predetermined time period to form a first mixture.

Typically, the predetermined temperature is in the range of 90 to 110 °C and the predetermined time period is in the range of 25 to 35 minutes.

The first mixture is then cooled to room temperature to form a cooled mixture. In an embodiment of the present disclosure, the second mixture is cooled till it attains a temperature of 25 to 30 °C.

The cooled mixture is then subjected to centrifugation in an environment maintained at 4 °C until the cooled mixture separates into a supernatant and a residue. Typically, the centrifugation is carried out at a speed of 6000 to 10000 rpm for 20 to 40 minutes at 4 °C, to prevent further denaturation of protein and nucleotides, to obtain a supernatant and a residue. The supernatant is then separated from the residue (residual cells and the cell debris) and concentrated to 1/4^{th} of its volume using concentrator at 4 °C to obtain the concentrated pharmacoactive nutrient medium.

In an embodiment of the present disclosure, the first mixture is maintained at a temperature in the range of 90 to 110 °C for 25 to 35 minutes.

In another embodiment of the present disclosure, the live marine algae is first washed with sterile water at least once and then added in the reaction vessel. Typically, the live marine algae is *Chlorella vuglaris.*

In still another embodiment of the present disclosure, the separation of the supernatant is carried out by at least one method selected from the group consisting of, but not limited to, decantation and filtration.

In yet another embodiment of the present disclosure, the pharmacoactive nutrient medium is substantially devoid of heavy metals.

In still another embodiment of the present disclosure, the ratio of water and live marine algae is in the range of 100:10 to 100:15.

The present disclosure is further described in light of the following experiments which are set forth for illustration purpose. The following experiments can be scaled up to industrial/commercial scale and the results obtained can be extrapolated to industrial scale.

### Experiments:

### Experiment 1: Preparation of pharmacoactive nutrient of the present disclosure

Pharmacoactive nutrient was prepared by using 2 different types of *Chlorella vuglaris* biomass; i.e., wet (live) biomass and dried (dead) biomass. Further the experiment was carried out in 2 different setups, i.e. the biomass was added to sterile water and then was heated to a suitable temperature and in a second scenario, the sterile water was first brought to boil and then the biomass was added to the boiling water. Thus 4 different sets of pharmacoactive nutrient were obtained.

Crude algal extract was prepared from C. *vulgaris* using the hot water extraction method. The cells during exponential phase were harvested by centrifugation at 4000 g for 10 min, and the pellet was washed thrice with sterile filtered water and the wet biomass was weighed.

**Process 1:** In a first reaction vessel, 100 g of wet biomass (*Chlorella vuglaris* biomass) was washed thrice first with sterile fresh water and was then added directly to 1000 mL of already boiling water (MilliQ water) and was allowed to boil for 15 minutes. After 15 minutes, the contents in the first reaction vessel were cooled to 28 °C and then centrifuged at 8000 rpm for 30 min at 4 °C. The supernatant (extract) obtained was collected and, concentrated to 1/4^{th} of its volume using concentrator at 4 °C to obtain a concentrate extract and was labeled as W-HWE I (Wet - hot water extracts).

**Process 2:** In a second reaction vessel, 100 g of wet biomass (*Chlorella vuglaris* biomass) was washed thrice first with sterile fresh water and was then added to 1000 mL of water (MilliQ water) and was gradually heated to 100 °C for 30 minutes. After 30 minutes, the contents in the first reaction vessel were cooled to 28 °C and then centrifuged at 8000 rpm for 30 min at 4 °C. The supernatant (extract) obtained was collected and concentrated to 1/4^{th} of its volume using concentrator at 4°C and was labeled as W-HWE II (Wet - hot water extracts).

Correspondingly, two sets of 100 g of wet biomass were shade dried and subjected to the above said processes 1 and 2 with 1000 mL water, and the extracts were named as D-HWE I (Dried - hot water extracts) and D-HWE II (Dried - hot water extracts).

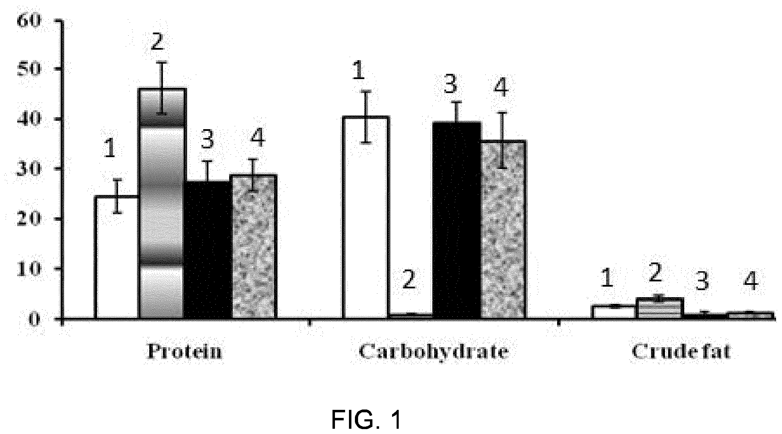

After analyzing the contents of the obtained supernatants, it was found that, the HWE (hot water extract) prepared from wet (live) biomass (*Chlorella vuglaris* biomass) of process 2 recorded significant level of protein (27.45%) and carbohydrates (39.21%) with 0.75% of crude fat, compared to the other extracts (as depicted in **Figure 1). Figure 1** illustrates macromolecular content of the hot water extracts (HWEs) of marine microalgae, *Chlorella vuglaris;* wherein 1 denotes W-HWE I; 2 denotes D-HWE I; 3 denotes W-HWE II; and 4 denotes D-HWE II.

The HWE from dry biomass (D-HWE I) although recorded more protein content (46.15 %) [as depicted in **Figure 1****],** the protein fractions in D-HWE I did not contain significant number of pharmaceutically vital proteins as compared to W-HWE II, thereby reducing its efficacy.

### Analysis of nutritional profile:

A nutritional profile for various pharmacoactive minerals, heavy metals, vitamins, free amino acids and free fatty acids present in the hot water extract from the wet (live) and dry biomass of marine *Chlorella vulgaris* was carried out and is depicted in **Table 1.** The HWEs were prepared using the above method, and the concentrated extracts were used for nutritional profile analysis. All the parameters were estimated following the standard protocol according to International standards, protein by AOAC 2001.11; total carbohydrates by AOAC 986.25; crude fat by IS 7874 (Part 1): 1975; energy using Bomb Calorimeter according to Nutritive value of Indian food (ICMR); calcium, phosphorous, potassium, sodium, magnesium, manganese and iron by SO-CHML-CTS-01-QU-063 using ICPOES; chloride by AOAC 950.52; vitamin B3 by SO-CHML-CTS-C01-QU-016 using HPLC; vitamins B5 and B12 by Ridascreen ELISA kit; free amino acid profile by SO-CHML-CTS-C01-QU-051; free fatty acids profile by ISO 5508:1990 & 5509:2000.

**TABLE 1. Level of minerals, heavy metals, vitamins, free amino acids and free fatty acids in the different HWEs of marine microalgae, Chlorellavulgaris**

| **Sr. No.** | **Parameters** | **W-HWE I** | **D-HWE I** | **W-HWE II** | **D-HWE II** |
|---|---|---|---|---|---|
| **Minerals** | | | | | |
| 1. | Calcium (mg/kg) | 154.80 | 26.02 | 58.34 | 30.39 |
| 2. | Phosphorous (mg/kg) | 53.75 | 55.22 | 39.95 | 65.70 |
| 3. | Potassium (mg/kg) | 145.42 | 97.43 | 112.31 | 118.64 |
| 4. | Sodium (mg/kg) | 200.91 | 152.22 | 140.41 | 184.25 |
| 5. | Magnesium (mg/kg) | 191.43 | 115.94 | 139.97 | 215.06 |
| 6. | Iron (mg/kg) | 13.30 | ND | 6.00 | 5.24 |
| 7. | Chloride (g/100 g) | <0.01 | <0.01 | <0.01 | 0.01 |

| **Heavy metals** | | | | | |
|---|---|---|---|---|---|
| 1. | Lead | ND | ND | ND | ND |
| 2. | Cadmium | ND | ND | ND | ND |
| 3. | Arsenic | ND | ND | ND | ND |
| 4. | Mercury | ND | ND | ND | ND |
| 5. | Manganese | ND | ND | ND | ND |

| **Vitamins** | | | | | |
|---|---|---|---|---|---|
| 1. | Vitamin B3 (mg/100g) | ND | ND | 0.29 | 0.31 |
| 2. | Vitamin B5 ((mg/100g) | 2.10 | 1.75 | 2.26 | 2.57 |
| 3. | Vitamin B12 (µg/kg) | 39.61 | 33.32 | 34.42 | 40.22 |

| **Free fatty acid profile (g/100g)** | | | | | |
|---|---|---|---|---|---|
| 1. | C16:0 Palmitic acid | <0.01 | 0.08 | 0.05 | <0.01 |
| 2. | C18:0 Stearic acid | <0.01 | 0.01 | 0.03 | <0.01 |
| 3. | C18:1 Oleic acid | <0.01 | 0.03 | 0.03 | <0.01 |
| 4. | Saturated fatty acids | <0.01 | 0.09 | 0.08 | <0.01 |
| 5. | Mono unsaturated fatty acids | <0.01 | 0.03 | 0.03 | <0.01 |
| 6. | Poly unsaturated fatty acids | <0.01 | <0.01 | 0.01 | <0.01 |
| 7. | Trans fatty acids | <0.01 | <0.01 | 0.01 | <0.01 |

| **Free amino acid profile (g/100g)** | | | | | |
|---|---|---|---|---|---|
| 1. | Aspartic acid | 0.01 | 0.02 | 0.02 | 0.02 |
| 2. | Serine | 0.00 | 0.01 | 0.01 | 0.01 |
| 3. | Glutamic acid | 0.01 | 0.02 | 0.04 | 0.04 |
| 4. | Glycine | 0.01 | 0.01 | 0.01 | 0.01 |
| 5. | Arginine | 0.01 | 0.01 | 0.02 | 0.02 |
| 6. | Threonine | 0.01 | 0.01 | 0.01 | 0.02 |
| 7. | Alanine | 0.04 | 0.02 | 0.04 | 0.04 |
| 8. | Proline | 0.04 | 0.05 | 0.04 | 0.07 |
| 9. | Cystine | 0.06 | 0.03 | 0.01 | 0.03 |
| 10. | Valine | 0.00 | 0.01 | 0.01 | 0.01 |
| 11. | Lysine | 0.01 | 0.01 | 0.05 | 0.05 |
| 12. | Isoleucine | 0.00 | 0.02 | 0.00 | 0.00 |
| 13. | Leucine | 0.00 | 0.01 | 0.01 | 0.01 |
| 14. | Phenylalanine | 0.00 | 0.00 | 0.00 | 0.01 |

| | | | | | |
|---|---|---|---|---|---|
| **ND = non-detectable** | | | | | |

Considerable amount of vitamins such as niacin (vitamin B3-0.29 mg/100g), pantothenic acid (vitamin B5-2.26 mg/100g) and cobalamin (vitamin B12-34.42 µg/kg) along with significant amount of minerals such as Ca (58.34 mg/kg), P (39.95 mg/kg), K (112.31 mg/kg) and Mg (139.97 mg/kg) were recorded in W-HWE II. Notable amount of amino acids such as glutamic acid (0.04 %), alanine (0.04 %), proline (0.04 %), lysine (0.05 %) and traces of other amino acids was also observed in W-HWE II, confirming its nutritional competence. Although the table determines that W-HWE I is significant in terms of minerals content, vitamin B3 was non-detectable in W-HWE I. **(Table 1).**

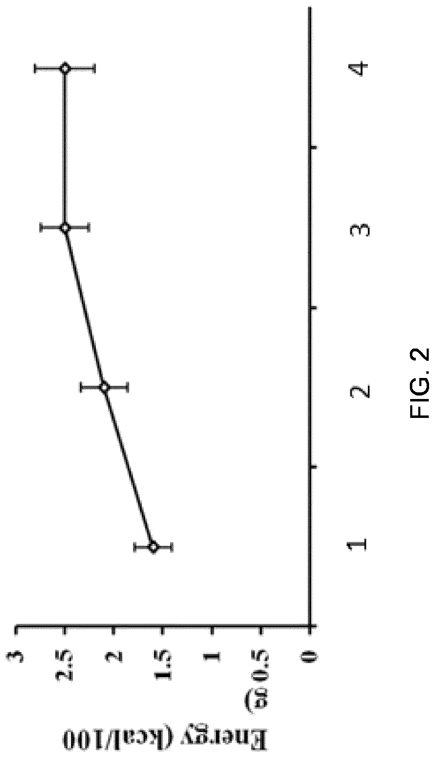

**Figure 2** illustrates the energy value of different HWEs of marine microalgae, *C*. *vulgaris,* wherein 1 denotes W-HWE I; 2 denotes D-HWE I; 3 denotes W-HWE II; and 4 denotes D-HWE II.The W-HWE II and D-HWE II possessed an energy value of 2.5 kcal/100 g, compared to W-HWE **I** (1.6 kcal/100 g) and D-HWE **I** (2.1 kcal/100 g). Thus, from the nutritional profile, it was concluded that the hot water extracts prepared from wet and dry biomass subjected to condition II was significant than the extracts prepared under condition I (as illustrated in **Figure 2****).**

### Experiment 2: Determination of the nature of proteins using Matrix-assisted laser desorption/ionization (MALDI)

The type of proteins present in the HWEs (W-HWE I, D-HWE I, W-HWE II and D-HWE II) was determined using high resolution MALDI-MS/MS (AB SCIEX QTRAP® 5500 System, Germany). The protein sample was digested and downsized with proteolytic enzyme into small peptides. These peptides were then separated based on their hydrophobicity in a reverse phase liquid chromatography column. Each chromatographic fraction was analyzed by Mass Spectrophotometer. By calculating the mass difference (mass-to-charge ratio) between adjacent y- or b- ions respectively, the peptide sequence was obtained as amino acids. The thermostability of the peptide fragments was generated from Uniprot database and the aliphatic index, which corresponds to the thermostability index of the protein, was determined using ProtParam tool of ExPASy. The complete profile of the proteins recorded in W-HWE I, D-HWE I, W-HWE II and D-HWE II are displayed below **(Table 2).**

**Table 2. Complete summary of the proteins present in the HWEs from wet and dry biomass of C. vulgaris along with its score and molecular mass**

| **Sr. No.** | **Database** | **Score** | **Mass** | **Description** |
|---|---|---|---|---|
| **Wet Biomass HWE Condition I (W-HWE I)** | | | | |
| 1. | Coccomyxa_subellipsoidea_C1 69_UniProt | 41 | 44680 | Kinesin-domain-containing protein |
| 2. | Coccomyxa_subellipsoidea_C1 69_UniProt | 39 | 24996 | HSP20-like chaperone |
| 4. | Coccomyxa_subellipsoidea_C1 69_UniProt | 30 | 148153 | Uncharacterized protein |
| 4. | Coccomyxa_subellipsoidea_C1 69_UniProt | 29 | 18037 | Peptidyl-prolylcis-trans isomerase |
| 5. | Coccomyxa_subellipsoidea_C1 69_UniProt | 26 | 8782 | Photosystem I iron-sulfur center |
| 6. | Coccomyxa_subellipsoidea_C1 69_UniProt | 21 | 49489 | Uncharacterized protein |
| 7. | Coccomyxa_subellipsoidea_C1 69_UniProt | 16 | 55227 | Pyrophosphate-fructose 6-phosphate 1-phosphotransferase beta-subunit |
| 8. | Coccomyxa_subellipsoidea_C1 69_UniProt | 16 | 120590 | Uncharacterized protein |
| 9. | Coccomyxa_subellipsoidea_C1 69_UniProt | 13 | 145620 | Uncharacterized protein |

| **Dry Biomass HWE Condition I (D-HWE I)** | | | | |
|---|---|---|---|---|
| 1. | Coccomyxa_subellipsoidea_C1 69_UniProt | 133 | 34291 | Polyubiquitin |
| 2. | Coccomyxa_subellipsoidea_C1 69_UniProt | 55 | 12046 | Uncharacterized protein |
| 3. | Coccomyxa_subellipsoidea_C1 69_UniProt | 49 | 20285 | GrpE protein homolog (Fragment) |
| 4. | Coccomyxa_subellipsoidea_C1 69_UniProt | 48 | 24996 | HSP20-like chaperone |
| 5. | Coccomyxa_subellipsoidea_C1 69_UniProt | 47 | 39531 | Photosystem II D2 protein |
| 6. | Coccomyxa_subellipsoidea_C1 69_UniProt | 41 | 8782 | Photosystem I iron-sulfur center |
| 7. | Coccomyxa_subellipsoidea_C1 69_UniProt | 40 | 44680 | Kinesin-domain-containing protein |
| 8. | Coccomyxa_subellipsoidea_C1 69_UniProt | 25 | 40074 | Isocitrate dehydrogenase, NAD-dependent |
| 9. | Coccomyxa_subellipsoidea_C1 69_UniProt | 23 | 18037 | Peptidyl-prolylcis-trans isomerase |
| 10. | Coccomyxa_subellipsoidea_C1 69_UniProt | 21 | 53762 | Pre-mRNA-splicing factor |
| 11. | Coccomyxa_subellipsoidea_C1 69_UniProt | 19 | 68824 | Heat shock protein 70 |
| 12. | Coccomyxa_subellipsoidea_C1 69_UniProt | 17 | 68674 | Uncharacterized protein |

| **Wet Biomass HWE Condition II (W-HWE II)** | | | | |
|---|---|---|---|---|
| 1. | SwissProt | 713 | 14691 | Ubiquitin-60S ribosomal protein L40 |
| 2. | SwissProt | 299 | 39221 | Photosystem II D2 protein |
| 3. | Coccomyxa_subellipsoidea_C1 69_UniProt | 192 | 71786 | Heat shock protein 70 |
| 4. | SwissProt | 113 | 75469 | Stromal 70 kDa heat shock-related protein, chloroplastic |
| 5. | Coccomyxa_subellipsoidea_C1 69_NCBI | 84 | 68824 | Heat shock protein |
| 6. | Coccomyxa_subellipsoidea_C1 69_UniProt | 164 | 35752 | Malate dehydrogenase |
| 7. | Coccomyxa_subellipsoidea_C1 69_UniProt | 148 | 11981 | Thioredoxin |
| 8. | Coccomyxa_subellipsoidea_C1 69_UniProt | 114 | 18037 | Peptidyl-prolylcis-trans isomerase |
| 9. | SwissProt | 113 | 38125 | Photosystem Q(B) protein |
| 10. | SwissProt | 94 | 42493 | S-adenosylmethionine synthase 1 |
| 11. | SwissProt | 85 | 13439 | 50S ribosomal protein L12, chloroplastic |
| 12. | Coccomyxa_subellipsoidea_C1 69_UniProt | 85 | 20285 | GrpE protein homolog (Fragment) |
| 13. | Coccomyxa_subellipsoidea_C1 69_UniProt | 81 | 16922 | EF-hand |
| 14. | SwissProt | 78 | 29709 | 40S ribosomal protein S3a |
| 15. | Coccomyxa_subellipsoidea_C1 69_UniProt | 74 | 38622 | Fructose-bisphosphatealdolase |
| 16. | SwissProt | 73 | 54675 | ATP synthase subunit alpha, chloroplastic |
| 17. | Coccomyxa_subellipsoidea_C1 69_UniProt | 72 | 24996 | HSP20-like chaperone |
| 18. | SwissProt | 69 | 11950 | Cytochrome c |
| 19. | Coccomyxa_subellipsoidea_C1 69_UniProt | 67 | 8195 | Small nuclear ribonucleo protein polypeptide G |
| 20. | SwissProt | 56 | 36663 | ADP, ATP carrier protein |
| 21. | Swissprot | 55 | 15947 | Histone H2B |
| 22. | SwissProt | 52 | 37280 | Fructose-1,6-bisphosphatase, cytosolic |
| 23. | Coccomyxa_subellipsoidea_C1 69_UniProt | 51 | 121492 | AAA-domain-containing protein |
| 24. | Coccomyxa_subellipsoidea_C1 69_UniProt | 51 | 55302 | ATP synthase subunit alpha |
| 25. | SwissProt | 48 | 25274 | GTP-binding nuclear protein Ran/TC4 |
| 26. | SwissProt | 48 | 56897 | Serine hydroxymethyltransferase 2, mitochondrial |
| 27. | Coccomyxa_subellipsoidea_C1 69_UniProt | 48 | 61245 | Aldehyde dehydrogenase |
| 28. | Coccomyxa_subellipsoidea_C1 69_UniProt | 47 | 66121 | SM/Sec1-family protein |
| 29. | Coccomyxa_subellipsoidea_C1 69_UniProt | 47 | 59677 | MCM-domain-containing protein |
| 30. | Coccomyxa_subellipsoidea_C1 69_UniProt | 44 | 12367 | Histone H2B (Fragment) |
| 31. | Coccomyxa_subellipsoidea_C1 69_UniProt | 39 | 119061 | ARM repeat-containing protein |
| 32. | Coccomyxa_subellipsoidea_C1 69_UniProt | 38 | 202236 | Uncharacterized protein |
| 33. | SwissProt | 38 | 55542 | Probable 26S proteasome non-ATPase regulatory subunit 3 |
| 34. | Coccomyxa_subellipsoidea_C1 69_UniProt | 38 | 26613 | Uncharacterized protein |
| 35. | Coccomyxa_subellipsoidea_C1 69_UniProt | 37 | 74579 | Uncharacterized protein |
| 36. | Coccomyxa_subellipsoidea_C1 69_UniProt | 37 | 108391 | Calcium-transporting ATPase |
| 37. | Coccomyxa_subellipsoidea_C1 69_UniProt | 36 | 88409 | Uncharacterized protein |
| 38. | SwissProt | 35 | 34106 | Hydroxyphenylpyruvateredu ctase |
| 39. | Coccomyxa_subellipsoidea_C1 69_UniProt | 35 | 68925 | Uncharacterized protein |
| 40. | Coccomyxa_subellipsoidea_C1 69_UniProt | 34 | 44680 | Kinesin-domain-containing protein |
| 41. | SwissProt | 34 | 20670 | 60S ribosomal protein L11 |
| 42. | Coccomyxa_subellipsoidea_C1 69_UniProt | 33 | 111229 | Uncharacterized protein |
| 43. | Coccomyxa_subellipsoidea_C1 69_UniProt | 33 | 118425 | Uncharacterized protein |
| 44. | SwissProt | 33 | 61127 | Maturase K |
| 45. | Coccomyxa_subellipsoidea_C1 69_UniProt | 32 | 95448 | ARM repeat-containing protein |
| 46. | Coccomyxa_subellipsoidea_C1 69_UniProt | 32 | 40938 | Magnesium-chelatase subunit |
| 47. | Coccomyxa_subellipsoidea_C1 69_UniProt | 32 | 17950 | Eukaryotic initiation factor |
| 48. | Coccomyxa_subellipsoidea_C1 69_UniProt | 32 | 15938 | UBC-like protein |
| 49. | Coccomyxa_subellipsoidea_C1 69_UniProt | 31 | 143896 | Putative pleiotropic drug resistance protein 3 |
| 50. | Coccomyxa_subellipsoidea_C1 69_UniProt | 31 | 38257 | Kinesin-domain-containing protein |
| 51. | SwissProt | 31 | 89275 | Cell division cycle protein 48 homolog |
| 52. | SwissProt | 30 | 65752 | Alpha-farnesene synthase |
| 53. | Coccomyxa_subellipsoidea_C1 69_UniProt | 30 | 40236 | Pkinase-domain-containing protein |
| 54. | Coccomyxa_subellipsoidea_C1 69_UniProt | 30 | 145620 | Uncharacterized protein |
| 55. | SwissProt | 29 | 64910 | 4-alpha-glucanotransferase, chloroplastic/amyloplastic |
| 56. | SwissProt | 28 | 46457 | Floricaula/leafy-like protein FL1 |
| 57. | SwissProt | 28 | 43215 | Probable caffeine synthase 4 |
| 58. | SwissProt | 28 | 68792 | V-type proton ATPase catalytic subunit A |
| 59. | SwissProt | 28 | 20885 | 50S ribosomal protein L5, plastid |
| 60. | Coccomyxa_subellipsoidea_C1 69_UniProt | 28 | 53237 | Dihydrolipoyl dehydrogenase |
| 61. | Coccomyxa_subellipsoidea_C1 69_UniProt | 28 | 96682 | Uncharacterized protein |
| 62. | SwissProt | 28 | 55618 | Obtusifoliol 14-alpha demethylase |
| 63. | SwissProt | 27 | 225288 | Putative membrane protein ycf1 |
| 64. | Coccomyxa_subellipsoidea_C1 69_UniProt | 27 | 38765 | Cyclophilin-like protein |
| 65. | SwissProt | 26 | 244798 | Protein Ycf2 |
| 66. | Coccomyxa_subellipsoidea_C1 69_UniProt | 26 | 42529 | Kinase-like protein |
| 67. | Coccomyxa_subellipsoidea_C1 69_UniProt | 26 | 35713 | Ubiquinone biosynthesis protein COQ9 |
| 68. | SwissProt | 26 | 38558 | Lipoyl synthase, mitochondrial |
| 69. | SwissProt | 25 | 37041 | Thiamine thiazole synthase, chloroplastic |
| 70. | SwissProt | 25 | 13703 | 50S ribosomal protein L12, chloroplastic |
| 71. | Coccomyxa_subellipsoidea_C1 69_UniProt | 25 | 54803 | 26S proteasome regulatory subunit |
| 72. | SwissProt | 25 | 62434 | RuBisCO large subunit-binding protein subunit beta, chloroplastic |
| 73. | SwissProt | 25 | 46507 | Eukaryotic initiation factor 4A |
| 74. | SwissProt | 25 | 28543 | Oxygen-evolving enhancer protein 2-2, chloroplastic |
| 75. | SwissProt | 25 | 23164 | 30S ribosomal protein S4, chloroplastic |
| 76. | Coccomyxa_subellipsoidea_C1 69_UniProt | 25 | 17167 | Uncharacterized protein |
| 77. | SwissProt | 24 | 33505 | Triosephosphateisomerase, chloroplastic |
| 78. | Coccomyxa_subellipsoidea_C1 69_UniProt | 24 | 42685 | Uncharacterized protein |
| 79. | Coccomyxa_subellipsoidea_C1 69_UniProt | 23 | 7450 | Uncharacterized protein |
| 80. | Coccomyxa_subellipsoidea_C1 69_UniProt | 23 | 47708 | Uncharacterized protein |
| 81. | SwissProt | 23 | 105023 | Protein translocase subunit SecA, chloroplastic |
| 82. | SwissProt | 23 | 104354 | Probable alanine--tRNA ligase, chloroplastic |
| 83. | SwissProt | 23 | 109115 | Phosphoenolpyruvate carboxylase 2 |
| 84. | SwissProt | 23 | 28687 | Inositol monophosphatase 2 |
| 85. | Coccomyxa_subellipsoidea_C1 69_UniProt | 22 | 75577 | Uncharacterized protein |
| 86. | SwissProt | 22 | 35123 | Guanine nucleotide-binding protein subunit beta-like protein |
| 87. | Coccomyxa_subellipsoidea_C1 69_UniProt | 22 | 152685 | Uncharacterized protein |
| 88. | Coccomyxa_subellipsoidea_C1 69_UniProt | 22 | 115624 | Uncharacterized protein |
| 89. | SwissProt | 22 | 8812 | Photosystem I iron-sulfurcenter |
| 90. | Coccomyxa_subellipsoidea_C1 69_UniProt | 22 | 31614 | Putative DnaJ protein |
| 91. | Coccomyxa_subellipsoidea_C1 69_UniProt | 22 | 49489 | Uncharacterized protein |
| 92. | SwissProt | 22 | 82417 | Photosystem I P700 chlorophyll a apoprotein A2 |
| 93. | Coccomyxa_subellipsoidea_C1 69_UniProt | 21 | 60457 | Acetohydroxy acid isomeroreductase |
| 94. | SwissProt | 21 | 68272 | 6(G)-fructosyltransferase |
| 95. | Coccomyxa_subellipsoidea_C1 69_UniProt | 21 | 98775 | Uncharacterized protein |
| 96. | Coccomyxa_subellipsoidea_C1 69_UniProt | 21 | 23585 | DUF924-domain-containing protein |
| 97. | Coccomyxa_subellipsoidea_C1 69_UniProt | 21 | 193044 | Uncharacterized protein |
| 98. | Coccomyxa_subellipsoidea_C1 69_UniProt | 21 | 25085 | Uncharacterized protein |
| 99. | Coccomyxa_subellipsoidea_C1 69_UniProt | 21 | 60441 | DEAD-domain-containing protein (Fragment) |
| 100. | SwissProt | 20 | 17741 | Polcalcin Jun o 2 |
| 101. | Coccomyxa_subellipsoidea_C1 69_UniProt | 20 | 52482 | Uncharacterized protein |
| 102. | Coccomyxa_subellipsoidea_C1 69_UniProt | 20 | 74787 | Isomerising glucosamine-fructose-6-phosphate aminotransferase |
| 103. | SwissProt | 20 | 5284 | Thionin |
| 104. | Coccomyxa_subellipsoidea_C1 69_UniProt | 20 | 51709 | Cytochrome P450 |
| 105. | SwissProt | 20 | 65147 | DELLA protein GAIP-B |
| 106. | Coccomyxa_subellipsoidea_C1 69_UniProt | 19 | 18783 | Uncharacterized protein |
| 107. | Coccomyxa_subellipsoidea_C1 69_UniProt | 19 | 40707 | Uncharacterized protein (Fragment) |
| 108. | Coccomyxa_subellipsoidea_C1 69_UniProt | 19 | 36263 | Brix-domain-containing protein |
| 109. | SwissProt | 19 | 56352 | Cytochrome P450 71B1 |
| 110. | Coccomyxa_subellipsoidea_C1 69_UniProt | 19 | 99190 | Kinase-like protein (Fragment) |
| 111. | Coccomyxa_subellipsoidea_C1 69_UniProt | 19 | 14570 | Uncharacterized protein |
| 112. | Coccomyxa_subellipsoidea_C1 69_UniProt | 19 | 72416 | Long-chain acyl-CoA synthetase 7 |
| 113. | Coccomyxa_subellipsoidea_C1 69_UniProt | 19 | 57457 | Putative phosphoribosylaminoimidaz olecarboxamide |
| 114. | SwissProt | 19 | 22680 | Auxin-induced protein 22E |
| 115. | Coccomyxa_subellipsoidea_C1 69_UniProt | 19 | 9933 | Ubiquitin-fold modifier 1 |
| 116. | SwissProt | 19 | 49196 | Glutamate-1-semialdehyde 2,1-aminomutase, chloroplastic |
| 117. | Coccomyxa_subellipsoidea_C1 69_UniProt | 19 | 102457 | Eukaryotic translation initiation factor 3 subunit C |
| 118. | Coccomyxa_subellipsoidea_C1 69_UniProt | 19 | 49603 | Uncharacterized protein |
| 119. | Coccomyxa_subellipsoidea_C1 69_UniProt | 18 | 16620 | Uncharacterized protein |
| 120. | Coccomyxa_subellipsoidea_C1 69_UniProt | 18 | 38641 | Uncharacterized protein |
| 121. | Coccomyxa_subellipsoidea_C1 69_UniProt | 17 | 28382 | Ferritin |
| 122. | Coccomyxa_subellipsoidea_C1 69_UniProt | 17 | 83743 | Uncharacterized protein |
| 123. | Coccomyxa_subellipsoidea_C1 69_UniProt | 16 | 141104 | Uncharacterized protein |
| 124. | SwissProt | 16 | 64635 | Isocitratelyase |
| 125. | SwissProt | 16 | 22161 | Soluble inorganic pyrophosphatase 2 |
| 126. | SwissProt | 16 | 42034 | Sedoheptulose-1,7-bisphosphatase, chloroplastic |
| 127. | Coccomyxa_subellipsoidea_C1 69_UniProt | 16 | 126749 | Uncharacterized protein |
| 128. | Coccomyxa_subellipsoidea_C1 69_UniProt | 15 | 68872 | Uncharacterized protein |
| 129. | SwissProt | 15 | 67812 | Formate--tetrahydrofolate ligase |
| 130. | Coccomyxa_subellipsoidea_C1 69_UniProt | 14 | 167691 | Uncharacterized protein |
| 131. | SwissProt | 14 | 39736 | Caffeic acid 3-O-methyltransferase |
| 132. | Coccomyxa_subellipsoidea_C1 69_UniProt | 13 | 64201 | 2-isopropylmalate synthase |

| **Dry biomass HWE Condition II (D-HWE II)** | | | | |
|---|---|---|---|---|
| 1. | SwissProt | 423 | 14691 | Ubiquitin-60S ribosomal protein L40 |
| 2. | Coccomyxa_subellipsoidea_C1 69_UniProt | 164 | 71786 | Heat shock protein 70 |
| 3. | Coccomyxa_subellipsoidea_C1 69_UniProt | 146 | 24996 | HSP20-like chaperone |
| 4. | SwissProt | 110 | 75469 | Stromal 70 kDa heat shock-related protein, chloroplastic |
| 5. | SwissProt | 104 | 38081 | Photosystem Q(B) protein |
| 6. | SwissProt | 97 | 39358 | Photosystem II D2 protein |
| 7. | SwissProt | 97 | 13439 | 50S ribosomal protein L12, chloroplastic |
| 8. | Coccomyxa_subellipsoidea_C1 69_UniProt | 91 | 18037 | Peptidyl-prolylcis-trans isomerase |
| 9. | Coccomyxa_subellipsoidea_C1 69_UniProt | 87 | 62675 | Chaperonin 60A |
| 10. | SwissProt | 80 | 29752 | 40S ribosomal protein S3a |
| 11. | Coccomyxa_subellipsoidea_C1 69_UniProt | 74 | 16922 | EF-hand |
| 12. | Coccomyxa_subellipsoidea_C1 69_UniProt | 73 | 55302 | ATP synthase subunit alpha |
| 13. | SwissProt | 69 | 55562 | ATP synthase subunit alpha, mitochondrial |
| 14. | SwissProt | 62 | 54675 | ATP synthase subunit alpha, chloroplastic |
| 15. | Coccomyxa_subellipsoidea_C1 69_UniProt | 72 | 20285 | GrpE protein homolog (Fragment) |
| 16. | Coccomyxa_subellipsoidea_C1 69_UniProt | 70 | 44680 | Kinesin-domain-containing protein |
| 17. | SwissProt | 70 | 25274 | GTP-binding nuclear protein Ran/TC4 |
| 18. | Coccomyxa_subellipsoidea_C1 69_UniProt | 68 | 295636 | Uncharacterized protein |
| 19. | Coccomyxa_subellipsoidea_C1 69_UniProt | 57 | 39518 | Dihydrolipoamidesuccinyltra nsferase |
| 20. | Coccomyxa_subellipsoidea_C1 69_UniProt | 55 | 28344 | Pyrroline-5-carboxylate reductase |
| 21. | SwissProt | 44 | 39073 | Glyceraldehyde-3-phosphate dehydrogenase |
| 22. | Coccomyxa_subellipsoidea_C1 69_UniProt | 50 | 45610 | Elongation factor Tu |
| 23. | Coccomyxa_subellipsoidea_C1 69_UniProt | 48 | 12046 | Uncharacterized protein |
| 24. | SwissProt | 47 | 16214 | Probable histone H2B.1 |
| 25. | SwissProt | 44 | 44863 | Elongation factor Tu, chloroplastic |
| 26. | Coccomyxa_subellipsoidea_C1 69_UniProt | 44 | 8248 | Photosystem I reaction centre subunit IV/PsaE |
| 27. | Coccomyxa_subellipsoidea_C1 69_UniProt | 42 | 30041 | Uncharacterized protein |
| 28. | SwissProt | 41 | 62434 | RuBisCO large subunit-binding protein subunit beta, chloroplastic |
| 29. | SwissProt | 41 | 42096 | Phosphoglycerate kinase, cytosolic |
| 30. | SwissProt | 41 | 57443 | Cyanidin 3-O-glucoside 5-O-glucosyltransferase (acyl-glucose) |
| 31. | Coccomyxa_subellipsoidea_C1 69_UniProt | 40 | 7374 | Uncharacterized protein |
| 32. | SwissProt | 40 | 35644 | Guanine nucleotide-binding protein subunit beta-like protein |
| 33. | SwissProt | 42 | 116969 | Putative membrane protein ycf1 (Fragment) |
| 34. | SwissProt | 40 | 54628 | 1-aminocyclopropane-1-carboxylate synthase 2 |
| 35. | Coccomyxa_subellipsoidea_C1 69_UniProt | 39 | 10672 | Small molecular heat shock protein 10 |
| 36. | Coccomyxa_subellipsoidea_C1 69_UniProt | 39 | 69721 | MFS general substrate transporter |
| 37. | SwissProt | 38 | 510343 | Dynein-1-beta heavy chain, flagellar inner arm I1 complex |
| 38. | SwissProt | 37 | 54415 | Legumin type B |
| 39. | Coccomyxa_subellipsoidea_C1 69_UniProt | 37 | 18769 | Uncharacterized protein |
| 40. | SwissProt | 37 | 13703 | 50S ribosomal protein L12, chloroplastic |
| 41. | SwissProt | 35 | 37280 | Fructose-1,6-bisphosphatase, cytosolic |
| 42. | Coccomyxa_subellipsoidea_C1 69_UniProt | 35 | 70200 | Uncharacterized protein |
| 43. | SwissProt | 34 | 89714 | Cell division cycle protein 48 homolog |
| 44. | SwissProt | 34 | 27190 | Alpha-amylase inhibitor 1 |
| 45. | SwissProt | 34 | 23641 | 60S ribosomal protein L13-2 |
| 46. | Coccomyxa_subellipsoidea_C1 69_UniProt | 34 | 246245 | Uncharacterized protein |
| 47. | SwissProt | 32 | 36663 | ADP,ATP carrier protein |
| 48. | Coccomyxa_subellipsoidea_C1 69_UniProt | 31 | 139520 | Uncharacterized protein |
| 49. | Coccomyxa_subellipsoidea_C1 69_UniProt | 31 | 63328 | Uncharacterized protein |
| 50. | Coccomyxa_subellipsoidea_C1 69_UniProt | 31 | 90617 | Uncharacterized protein |
| 51. | Coccomyxa_subellipsoidea_C1 69_UniProt | 31 | 49489 | Uncharacterized protein |
| 52. | Coccomyxa_subellipsoidea_C1 69_UniProt | 31 | 18783 | Uncharacterized protein |
| 53. | Coccomyxa_subellipsoidea_C1 69_UniProt | 31 | 17950 | Eukaryotic initiation factor |
| 54. | Coccomyxa_subellipsoidea_C1 69_UniProt | 31 | 146919 | Uncharacterized protein |
| 55. | SwissProt | 30 | 43215 | Probable caffeine synthase 4 |
| 56. | SwissProt | 30 | 28756 | Chlorophyll a-b binding protein 4, chloroplastic |
| 57. | SwissProt | 30 | 87187 | Beta-amyrin synthase |
| 58. | Coccomyxa_subellipsoidea_C1 69_UniProt | 30 | 121492 | AAA-domain-containing protein |
| 59. | Coccomyxa_subellipsoidea_C1 69_UniProt | 30 | 55356 | MFS general substrate transporter |
| 60. | Coccomyxa_subellipsoidea_C1 69_UniProt | 29 | 39299 | Uncharacterized protein |
| 61. | Coccomyxa_subellipsoidea_C1 69_UniProt | 29 | 40046 | RNA-binding domain-containing protein |
| 62. | Coccomyxa_subellipsoidea_C1 69_UniProt | 29 | 81552 | Uncharacterized protein |
| 63. | SwissProt | 29 | 55618 | Obtusifoliol 14-alpha demethylase |
| 64. | Coccomyxa_subellipsoidea_C1 69_UniProt | 28 | 40707 | Uncharacterized protein (Fragment) |
| 65. | Coccomyxa_subellipsoidea_C1 69_UniProt | 28 | 71764 | SBP-domain-containing protein |
| 66. | Coccomyxa_subellipsoidea_C1 69_UniProt | 28 | 85027 | DNA polymerase |
| 67. | Coccomyxa_subellipsoidea_C1 69_UniProt | 28 | 114798 | E1 subunit of 2-oxoglutarate dehydrogenase |
| 68. | Coccomyxa_subellipsoidea_C1 69_UniProt | 28 | 198054 | Uncharacterized protein |
| 69. | Coccomyxa_subellipsoidea_C1 69_UniProt | 28 | 287211 | Uncharacterized protein |
| 70. | SwissProt | 27 | 26720 | Triosephosphateisomerase, cytosolic |
| 71. | Coccomyxa_subellipsoidea_C1 69_UniProt | 27 | 133590 | Uncharacterized protein |
| 72. | SwissProt | 26 | 96500 | DNA-directed RNA polymerase subunit beta' |
| 73. | SwissProt | 26 | 17329 | Eukaryotic translation initiation factor 5A |
| 74. | SwissProt | 26 | 57884 | Trans-cinnamate 4-monooxygenase |
| 75. | SwissProt | 26 | 68272 | 6(G)-fructosyltransferase |
| 76. | Coccomyxa_subellipsoidea_C1 69_UniProt | 26 | 27365 | tRNApseudouridine synthase (Fragment) |
| 77. | SwissProt | 26 | 158476 | DNA-directed RNA polymerase subunit beta" |
| 78. | Coccomyxa_subellipsoidea_C1 69_UniProt | 26 | 118425 | Uncharacterized protein |
| 79. | Coccomyxa_subellipsoidea_C1 69_UniProt | 26 | 78888 | Uncharacterized protein |
| 80. | Coccomyxa_subellipsoidea_C1 69_UniProt | 25 | 61918 | Uncharacterized protein |
| 81. | SwissProt | 25 | 21426 | 50S ribosomal protein L24, chloroplastic |
| 82. | Coccomyxa_subellipsoidea_C1 69_UniProt | 25 | 47765 | Elongation factor Ts, mitochondrial |
| 83. | Coccomyxa_subellipsoidea_C1 69_UniProt | 25 | 56130 | Uncharacterized protein |
| 84. | SwissProt | 24 | 4430 | 50S ribosomal protein L36, chloroplastic |
| 85. | Coccomyxa_subellipsoidea_C1 69_UniProt | 24 | 96970 | Uncharacterized protein |
| 86. | Coccomyxa_subellipsoidea_C1 69_UniProt | 24 | 61929 | Uncharacterized protein |
| 87. | SwissProt | 24 | 49196 | Glutamate-1-semialdehyde 2,1-aminomutase, chloroplastic |
| 88. | Coccomyxa_subellipsoidea_C1 69_UniProt | 23 | 27314 | Bax inhibitor-1 like-protein |
| 89. | SwissProt | 23 | 269246 | Protein Ycf2 |
| 90. | SwissProt | 22 | 60800 | Maturase K |
| 91. | Coccomyxa_subellipsoidea_C1 69_UniProt | 22 | 97968 | Kinase-like protein |
| 92. | SwissProt | 22 | 77684 | Phenylalanine ammonia-lyase |
| 93. | Coccomyxa_subellipsoidea_C1 69_UniProt | 21 | 49811 | Uncharacterized protein |
| 94. | Coccomyxa_subellipsoidea_C1 69_UniProt | 21 | 84349 | COP-II coat subunit |
| 95. | SwissProt | 21 | 17537 | 30S ribosomal protein S7, chloroplastic |
| 96. | Coccomyxa_subellipsoidea_C1 69_UniProt | 21 | 34673 | Uncharacterized protein |
| 97. | SwissProt | 21 | 5284 | Thionin |
| 98. | Coccomyxa_subellipsoidea_C1 69_UniProt | 21 | 77376 | Histone H3 K4-specific methyltransferase SET7/9 N-terminal domain-containing protein |
| 99. | SwissProt | 20 | 13602 | 50S ribosomal protein L14, chloroplastic |
| 100. | SwissProt | 20 | 64063 | Pectinesterase/pectinestera se inhibitor U1 |
| 101. | Coccomyxa_subellipsoidea_C1 69_UniProt | 20 | 36918 | Uncharacterized protein |
| 102. | Coccomyxa_subellipsoidea_C1 69_UniProt | 20 | 38765 | Cyclophilin-like protein |
| 103. | Coccomyxa_subellipsoidea_C1 69_UniProt | 20 | 42685 | Uncharacterized protein |
| 104. | SwissProt | 19 | 252375 | DNA-directed RNA polymerase subunit beta |
| 105. | SwissProt | 19 | 12646 | MFS14 protein |
| 106. | SwissProt | 19 | 57256 | Serine hydroxymethyltransferase, mitochondrial |
| 107. | Coccomyxa_subellipsoidea_C1 69_UniProt | 18 | 50556 | PLP-dependent transferase (Fragment) |
| 108. | Coccomyxa_subellipsoidea_C1 69_UniProt | 18 | 28788 | Uncharacterized protein |
| 109. | Coccomyxa_subellipsoidea_C1 69_UniProt | 18 | 31445 | Uncharacterized protein |
| 110. | Coccomyxa_subellipsoidea_C1 69_UniProt | 18 | 129205 | Lipoxygenase (Fragment) |
| 111. | Coccomyxa_subellipsoidea_C1 69_UniProt | 18 | 70627 | RNI-like protein |
| 112. | Coccomyxa_subellipsoidea_C1 69_UniProt | 18 | 19183 | 50S ribosomal protein L35 |
| 113. | SwissProt | 18 | 4565 | Photosystem I reaction center subunit IX |
| 114. | Coccomyxa_subellipsoidea_C1 69_UniProt | 18 | 35543 | Uncharacterized protein |
| 115. | Coccomyxa_subellipsoidea_C1 69_UniProt | 18 | 39097 | Kinase-like protein (Fragment) |
| 116. | SwissProt | 18 | 199686 | Putative membrane protein ycf1 |
| 117. | SwissProt | 17 | 24069 | Probable 1-Cys peroxiredoxin |
| 118. | Coccomyxa_subellipsoidea_C1 69_UniProt | 17 | 69247 | Uncharacterized protein |
| 119. | SwissProt | 17 | 88286 | Dammarenediol II synthase |
| 120. | SwissProt | 17 | 29106 | Endochitinase A |
| 121. | Coccomyxa_subellipsoidea_C1 69_UniProt | 16 | 99594 | Alpha-1,6-glucosidase (Fragment) |
| 122. | Coccomyxa_subellipsoidea_C1 69_UniProt | 16 | 36398 | Glycosyltransferase (Fragment) |
| 123. | SwissProt | 15 | 87466 | Germanicol synthase |
| 124. | Coccomyxa_subellipsoidea_C1 69_UniProt | 14 | 119549 | Transcription elongation factor SPT5 |
| 125. | SwissProt | 14 | 17189 | Pathogenesis-related protein STH-21 |
| 126. | Coccomyxa_subellipsoidea_C1 69_UniProt | 13 | 113404 | Antiviral helicase |

The results in the above **Table 2** illustrate about 9 proteins in W-HWE I, 12 proteins in D-HWE I, 132 proteins in W-HWE II and 126 proteins in D-HWE II. Proteins such as kinesin-domain containing protein, heat shock protein (HSP20)-like chaperone, peptidyl-prolylcis-trans isomerase, photosystem I iron-sulfur center and pyrophosphate-fructose 6-phosphate 1-phosphotransferase beta subunit was recorded in W-HWE I.

In D-HWE I, proteins such as polyubiquitin, GrpE protein homolog (Fragment), photosystem II D2 protein, photosystem I iron-sulfur center, kinesin-domain-containing protein, isocitrate dehydrogenase, NAD-dependent, peptidyl-prolylcis-trans isomerase, pre-mRNA-splicing factor and heat shock proteins such as (HSP20 like chaperone and HSP70) were documented in D-HWE I.

An assortment of heat shock proteins (HSP) [HSP70 and HSP20 like chaperone], photosynthesis related proteins (photosystem II D2 protein, photosystem Q(B) protein, photosystem I iron sulfur center, photosystem I P700 chlorophyll a apoprotein A2 and RUBISCO large subunit binding protein), proteins involved in translation (50S ribosomal protein L5, 12, Ubiquitin-60S ribosomal protein L40, L11, 30S ribosomal protein S4, 40S ribosomal protein S3a, Elongation Factor (EF-hand), 26S proteasome regulatory subunit, probable 26S proteasome non-ATPase regulatory subunit 3, eukaryotic initiation factor 4A), etc. were documented in W-HWE II.

Numerous enzymes which catalyze key biological reactions, such as S-adenosyl methionine sysnthase 1, peptidyl-prolylcis-trans isomerase, malate dehydrogenase, fructose-bisphosphatealdolase, ATP synthase, fructose 1,6-bisphosphatase, serine hydroxyl methyltransferase 2, aldehyde dehydrogenase, calcium-transporting ATPase, hydroxyphenyl pyruvate reductase, maturase K, magnesium-chelatase, alpha-farnesene synthase, 4-alpha-glucanotransferase, probable caffeine synthase 4, dihydrolipoyl dehydrogenase, V-type proton ATPase, obtusifoliol 14-alpha demethylase, lipoyl synthase, thiamine thiazole synthase, triosephosphateisomerase, protein translocase subunit SecA, probable alanine-tRNA ligase, phosphoenolpyruvate carboxylase 2, inositol monophosphatase 2, acetohydroxy acid isomeroreductase, 6(G)-fructosyltransferase, isomerising glucosamine-fructose-6-phosphate aminotransferase, glutamate-1-semialdehyde 2,1-aminomutase, isocitratelyase, soluble inorganic pyrophosphatase 2, sedoheptulose 1,7-bisphosphatase, formate-tetrahydrofolate ligase, caffeic acid 3-o-methyltransferase, 2-isopropylmalate synthase, etc. were also documented in W-HWE II.

Certain biologically vital proteins, such as thioredoxin, GrpE protein homolog, cytochrome c (cytochrome P450, cytochrome P450 71B1), small nuclear ribonucleoprotein polypeptide G, ADP, ATP carrier protein, Histone H2B, AAA-domain containing protein, GTP-binding nuclear protein Ran/TC4, SM/Sec1-family protein, MCM-domain containing protein, ARM repeat containing protein, kinesin domain containing protein, UBC-like protein, cell division cycle protein 48 homolog, floricaula/leafy-like protein FL1, putative membrane protein ycf1, cyclophilin-like protein, protein Ycf2, ubiquinone biosynthesis protein COQ9, oxygen-evolving enhancer protein2, guanine nucleotide-binding protein, putative DNAJ protein, DUF924-domain containing protein, DEAD-domain containing protein, polcalin Jun O2, thionin, DELLA protein GAIP-B, Brix-domain containing protein, putative phosphoribosyl amino imidazole carboxamide, auxin-induced protein 22E, ubiquitin-fold modifier, ferritin were also documented in W-HWE II.

D-HWE II also documented key enzymes such as peptidyl-prolylcis-trans isomerase, ATP synthase subunit alpha, dihydrolipoamidesuccinyltransferase, pyrroline-5-carboxylate reductase, glyceraldehydes-3-phosphate dehydrogenase, cyaniding 3-O-glucoside 5-O-glucosyltransferase, 1-aminocyclopropane-1 carboxylase synthase 2, fructose-1,6-bisphosphatase, alpha-amylase, probable caffeine synthase, beta-amyrin synthase, obtusifoliol 14-alpha demethylase, DNA polymerase, E1 subunit of 2-oxoglutarate dehydrogenase, triosephosphateisomerase, DNA-directed RNA polymerase subunit beta, trans-cinnamate 4-monooxygenase, 6(G)-fructosyltransferase, tRNApseudourdine synthase, glutamate-1-semialdehyde 2,1-aminomutase, maturase-k, phenylalanine, ammonia lyase, histone H3K4-specific methyltransferase SET7/9 N-terminal domain-containing protein, serine hydroxymethyltransferase, PLP-dependent transferase, lipoxygenase, dammarenediol II synthase, endochitinase A, alpha-1,6-glucosidase, glucosyltransferase, germanicol synthase and antiviral helicase.

Numerous other proteins such as ubiquitin-60S ribosomal protein L40, L13-2, 50S ribosomal protein Ts, L12, L14, L35, L36 and L24, 40S ribosomal protein S3a, 30S ribosomal protein S7, EF-hand, GrpE protein homolog, kinesin-domain containing protein, GTP-binding nuclear protein Ran/TC4, elongation factor Tu, probable histone H2B.1, guanine nucleotide-binding proteins subunit beta, putative membrane protein ycf1, MFS general substrate transporter, dynein-1-beta heavy chain, flagellar inner are I1 complex, legumin type B protein, cell division cycle protein 48 homolog, alpha amylase inhibitor 1, ADP, ATP carrier protein, eukaryotic initiation factor, AAA-domain containing protein, RNA binding domain containing protein, SBP domain containing protein, protein Ycf2, kinase-like protein, COP-II coat subunit, pectinesterase inhibitor, cyclophilin-like protein, probable 1-cys peroxiredoxin, transcription elongation factor SPT5 and pathogenesis-related protein STH21 were recorded in D-HWE II.

Accordingly, all the HWEs comprise a lot of uncharacterized proteins (4 in W-HWE I, 2 in D-HWE I, 32 in W-HWE II and 60 in D-HWE II). Further, these uncharacterized proteins remained as unclassified, suggesting that these could be putatively novel proteins.

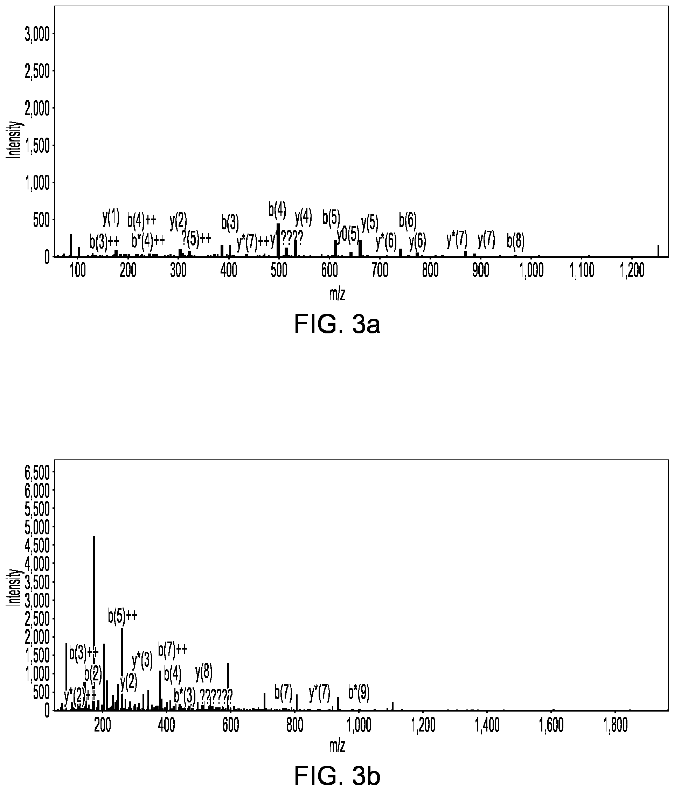

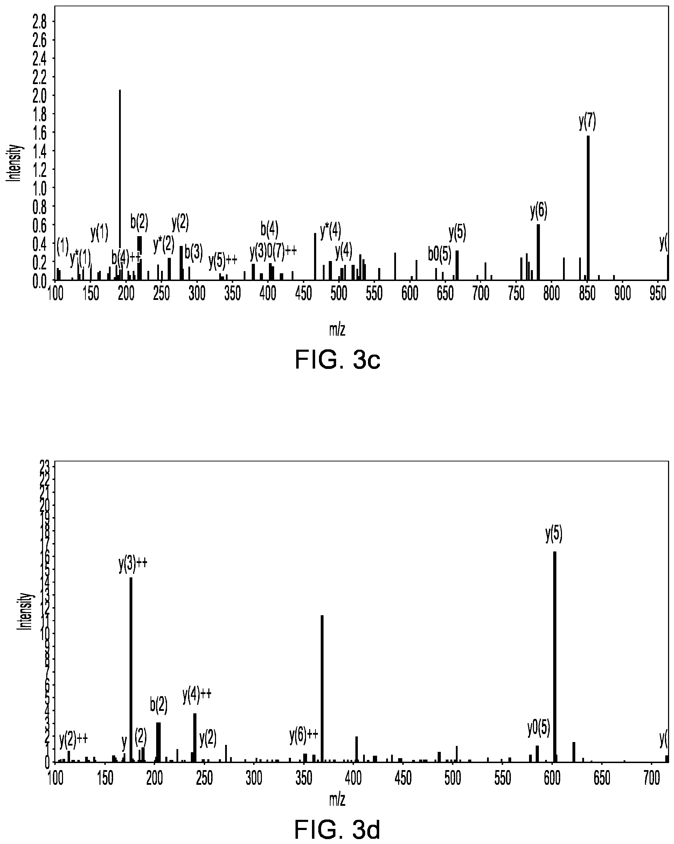

**Figures 3a & 3b** depicts Matrix-assisted laser desorption/ionization (MALDI) spectra of the wet and dry biomass HWEs of condition I. No pharmaceutically vital proteins were documented in W-HWE I and D-HWE I, although certain heat shock proteins and proteins involved in photosynthesis and carbohydrate metabolic pathway were observed. Hence, only the pharmaceutically important proteins in W-HWE II and D-HWE II were listed out in **Table 3.** An assortment of thermostable and pharmaceutically vital proteins such as thioredoxin, S-adenosylmethionine synthase-I, putative pleiotrophic drug resistance protein, dihydrolipoyl dehydrogenase, inositol monophosphatase-2 and Brix-domain containing protein were found in W-HWE II, compared to the dry biomass extracts (as illustrated in **Table 3** below and in **Figure 3c** and **Figure 3d**). **Figure 3c** illustrates the spectrum of W-HWE II, and **Figure 3d** illustrates the spectrum of D-HWE II. The wet biomass HWE of process II contains more potent proteins bearing significant activities such as cell growth, cell cycle regulation, defense, antioxidant, anti-cancer and proteins that are widely used in the treatment of depression, dementia, vacuolar myelopathy, liver injury, bipolar disorder, etc.

**TABLE 3. Pharmacologically vital proteins in the W-HWE II and D-HWE II along with its functions and aliphatic index**

| **Sr. No.** | **Name of the proteins** | **Function of the protein** | **Aliphatic index** |
|---|---|---|---|
| **Wet biomass HWE (W-HWE II)** | | | |
| 1. | Thioredoxin | Promotes cell growth at elevated levels and act as antioxidants | 93.00 |
| 2. | S-adenosylmethionine synthase 1 | Involved in gene transcription, cell proliferation, and production of secondary metabolites. Used against depression, dementia, vacuolar myelopathy, liver injury, migraine, osteoarthritis, and as a potential cancer chemo preventive agent | 82.97 |
| 3. | Putative pleiotropic drug resistance protein 3 | General defense protein | 102.02 |
| 4. | Dihydrolipoyl dehydrogenase (DLD) | Diaphorase activity of DLD may have an antioxidant role through its ability to scavenge nitric oxide and to reduce ubiquinone to ubiquinol | 89.34 |
| 5. | Inositol monophosphatase 2 | Has been implicated as the pharmacological target for lithium action in brain and useful in the treatment of bipolar disorder | 98.6 |
| 6. | Brix-domain-containing protein | Proteins from the Imp4/Brix superfamily appear to be involved in ribosomal RNA processing, which is essential for the functioning of all cells. | 82.78 |

| **Dry biomass HWE (D-HWE II)** | | | |
|---|---|---|---|
| 1. | Phenylalanine ammonia-lyase | In plants it is a key biosynthetic enzyme that catalyzes the first step in the synthesis of a variety of polyphenyl compounds and is mainly involved in defense mechanisms. | 91.68 |
| 2. | Pathogenesis-related protein STH-21 | Defense mechanism | 89.23 |
| 3. | Antiviral helicase | Acts as anti-cancer and anti-viral target | 88.57 |
| 4. | Endochitinase A | Defense against chitin containing fungal pathogens | 59.07 |

Comparison of the nutritional value, energy value as well as the number of pharmacologically important proteins in the various HWEs concluded that W-HWE II is the most effective extract with maximum nutritional value and pharmacological potency. Hence, this particular extract, W-HWE II prepared from the live biomass of marine microalgae by gradual heating to 100 °C for 30 min, holds maximum nutritional and pharmaceutical value.

### Experiment 3: Preparation of f/2 media comprising W-HWE II for algal growth enhancement.

About, 10% of the inoculums (*C*. *vulgaris*) was added into the f/2 medium containing the standard constituents, as shown below in **Table 4** (Guillard and Ryther, 1962). 1% W-HWE II was also added to 1 L of f/2 culture medium.

**Table 4: f/2 media (Guillard and Ryther, 1962)**

| **f/2 Media constituents** | **Quantity per litre** |
|---|---|
| NaNO₃ (75.0 g/L dH₂O) | 1 mL |
| NaH₂PO₄·H₂O (5.0 g/L dH₂O) | 1 mL |
| f/2 Trace Metal Solution | 1 mL |
| It comprises of the following constituents: | |
| FeCl3.6H2O (3.15 g/L) | |
| Na2EDTA.2H2O (4.36 g/L) | |
| CuSO4.5H2O (9.8 g/L) - 1 mL | |
| Na2MoO4.2H2O (6.3 g/L) - 1 mL | |
| ZnSO4.7H2O (22 g/L) - 1 mL | |
| CoCl2.6H2O (10 g/L) - 1 mL | |
| MnCl2.4H2O (180 g/L) - 1 mL | |
| f/2 Vitamin Solution | 0.5 mL |
| It comprises of the following constituents: | |
| Thiamine HCl (200 mg) | |
| Biotin (1 g/L)-1 mL | |
| Cyanocobalamin (1 g/L) - 1 mL | |
| W-HWE II (1%-1g/L) | 1 mL |

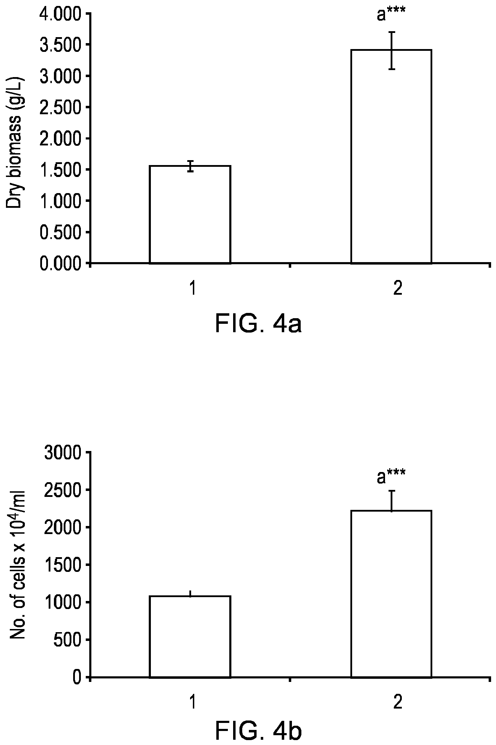

The culture was allowed to grow at 24 ± 1 °C at 18h: 6h (Light/Dark regime) with a light intensity of 200-400 µmol m⁻² s⁻¹. Algal biomass production was estimated using the method of Zhu & Lee (1997) and cell count using haemocytometer. The dry weight biomass estimation was carried out on the 15^{th} day from both the control (without the addition of W-HWE II) and W-HWE II added culture. Maximum biomass production (3.42 g/L) was achieved in W-HWE II added culture as compared to control (1.560 g/L), as illustrated in **Figure 4a****,** wherein 1 denotes control and 2 denotes W-HWE II.

As depicted in **Figure 4b** (wherein 1 denotes control and2 denotes W-HWE II), the cell count in the medium containing W-HWE II also revealed a significant increase (2197 x 10⁴ cells/mL) as compared to the number of cells in the medium without W-HWE II (1058 x 10⁴ cells/mL).

### Experiment 4: Demonstration of the protective effect of W-HWE II against central nervous system depression.

### 4a. Animals:

Adult male albino mice weighing 25-30 g were used to study the central nervous system depressant activity. The animals were obtained from SreeVenkateshwara Enterprises Pvt. Ltd., Bangalore. All animals were housed for at least one week in the laboratory animal room prior to study. The selected animals (24 numbers) were housed in polypropylene cages in the standard environmental conditions of temperature of 22 ± 3°C and 45-60% relative humidity, 12:12 light: dark cycle and had free access to food and water. They were acclimatized to laboratory conditions before behavioral studies.

### 4b. Preparation of drug solution:

The pharmacoactive nutrient from W-HWE II, which was found to be an effective extract among other extracts (D-HWE I, W-HWE II and D-HWE II), was freeze dried to obtain dry powder. The dry powder of the extract was dissolved in distilled water and administered orally to the mice at two different concentrations, 25 mg/kg/day and 50 mg/kg/day. A group receiving only vehicle, distilled water was taken as the vehicle control group. The central nervous system depressant drug, phenobarbitone (PB), was used in the present study to induce depression in the central nervous system at a concentration of 40 mg/kg b.w through intraperitoneal injection. The anti-depressant drug, chlorpromazine (CP) was used as a standard drug at a concentration of 5 mg/kg b.w, i.p. for comparing the potential of "W-HWE II".

### 4c. Phenobarbitone induced sleeping test in Albino mice:

Healthy Albino mice weighing 25-30 g were divided into four groups of six animals in each. Group I was maintained as normal control by administering the vehicle. Group II was treated with the standard drug, CP (5 mg/kg, i.p); Groups III and IV were treated orally with 25 mg/kg and 50 mg/kg W-HWE II. After 30 minutes of drug administrations, PB was administered intraperiotenally to all the groups at a dose of 40 mg/kg. The On Set of Sleeping (OS) time and Back of Time (BT) was noted in all the groups.

### 4d. Locomotor Activity using Actophotometer:

Healthy Albino mice weighing 25-30 g were divided into four groups of six animals in each as mentioned above. The photocell activity cage was utilized to determine the degree of depression. The actions of W-HWE II on spontaneous locomotor activity were measured automatically by using Actophotometer (Medicraft Photo Actometer, model No: 600-40, S. No: PA-0149, India). The units of the activity counts were arbitrary and based on the beam breaks by movement of the mice (Rabbani et al., 1995). The spontaneous locomotor activity of each mouse was recorded individually for 10 minutes using actophotometer. Basal activity score of all animals were noted before administration.

Phenobarbitone (PB-Sleeping dose) induced sleeping test was carried out, on mice, which indicated that the On Set Sleeping (OS) time significantly decreased in the W-HWE II administered mice at the concentration of 25 mg/kg (4.5 min) followed by W-HWE II at the concentration of 50 mg/kg (5.5 min), as compared to chlorpromazine (CP-antidepressant/antipsychotic drug) administered mice at a concentration of 5 mg/kg (6 min). Whereas, phenobarbitone alone administered mice exhibited an OS time of 10.25 min. Compared to phenobarbitone induced group (40.25 min), the standard antipsychotic drug, chlorpromazine (CP) exhibited a marginal increase in Back of Time (BT) by 50.5 min. Although, the W-HWE II administered mice revealed a trivial increase in Back of Time (BT) (44.25 min) at 50 mg/kg and 42.5 min at 25 mg/kg, considering along with OS time, it signifies a protective effect against central nervous system depression (as illustrated in **Figure 5a**). **Figure 5a** illustrates the sleeping test for assessing central nervous system depressant action; wherein 1 denotes on set sleeping; 2 denotes back of time; 3 denotes total duration; A denotes PB (40 mg/kg); B denotes PB (40 mg/kg) + CP (5 mg/kg); C denotes PB (40 mg/kg) + W-HWE II(25 mg/kg); and D denotes PB (40 mg/kg) + W-HWE II (50 mg/kg).

The present experiment also confirms the growth promoting ability of W-HWE II in mice model. The body weight of the animals during the experiment of PB-induced sleeping test revealed a significant increase in W-HWE II administered groups (30g weight at 25 mg/kg W-HWE II concentration and 28 g weight at 50 mg/kg), as compared to anti-psychotic drug, CP as well as depression inducing drug, PB administered animals within 3 days (as illustrated in **Figure 5b**). **Figure 5b** illustrates the body weight of mice during PB- induced central nervous system depression; wherein A denotes PB (40 mg/kg);B denotes PB (40 mg/kg) + CP (5 mg/kg); C denotes PB (40 mg/kg) + W-HWE II (25 mg/kg); and D denotes PB (40 mg/kg) + W-HWE II (50 mg/kg).

CP affects the body weight by reducing to 25.75 g from 26.75 g compared with PB stress induced mice within 3 days. Whereas, W-HWE II administration did not affect the body weight, but rather increased the body weight, thus suggesting its non toxic and growth promoting nature. Thus, W-HWE II has growth promoting activity and central nervous system, anti-depressant activity without any toxic side effects. W-HWE II also demonstrates neuropharmacological activity by reducing the locomotor activity in mice. **Table 5** illustrates the locomotor activity using Actophotometer which is measured as an index of alertness of mental activity, and hence a decrease in the motor activity as a result of diminished excitation in the central nervous system, may lead to calming and sedation. In the present experiment, the mice administered with W-HWE II demonstrated reduction in the motor activity (43.79% reduction at 25 mg/kg concentration and 67.61% reduction at 50 mg/kg concentration).

**Table 5.Locomotor activity using Actophotometer**

| **GROUPS** | **ACTOPHOTOMETER (sec/min)** | | **% reduction in motor activity** |
|---|---|---|---|
| | **Before** | **After** | |
| PB (40 mg/kg) | 233.5 ± 33.27 | 209 ± 15.6 | 10.49 |
| PB (40 mg/kg) + CP (5 mg/kg) | 226.3 ± 30.22 | 104.3 ± 10.14 | 53.91 (a*) |
| PB (40 mg/kg) + W-HWE II (25 mg/kg) | 190 ± 62.2 | 106.8 ± 14.12 | 43.79 (a*) |
| PB (40 mg/kg)+ W-HWE II(50mg/kg) | 355 ± 54.22 | 115 ± 12.77 | 67.61 (a*) |

Values are mean of six independent experiments ± S.D. Comparisons were made between a: PB vs. PB+CP, PB+W-HWE II (25 mg/kg) and PB+W-HWE II (50 mg/kg).
The symbol (*) represents the statistical significance at *p*< 0.05 (*).

### Experiment 5: Demonstration of the neuropharmacological activity of W-HWE I using Rota rod (muscular coordination) test:

Healthy Albino mice weighing 25-30 g were divided into four groups of six animals in each. Motor co-ordination test was conducted using a Rota Rod apparatus (Inco Ambala, India). The animals were placed on the moving rod prior to the treatment and the mice stayed on the rod without falling for 120 seconds were chosen for the study. The time animals take for falling from the rotating rod was noted before and after the treatment with extract (Kulkarni, 1987). The W-HWE II at both the doses (25 and 50 mg/kg) decreased the fall off time in mice, with 50 mg/kg being more significant, thus documenting its skeletal muscle relaxant property (as illustrated in **Table 6**). The potential muscle relaxant activity can be attributed to the muscle grip coupled with calming effect in mice, thereby supporting its neuropharmacological effect.

**Table 6. Muscle relaxant activity using rota-rod apparatus**

| GROUPS | Fall off time (sec/min) | | % reduction in fall off time |
|---|---|---|---|
| | Before | After | |
| PB (40 mg/kg) | 147 ± 12.4 | 124.5 ± 5.123 | 15.31 |
| PB (40 mg/kg) + CP (5 mg/kg) | 180 ± 0.1 | 112.8 ± 3.772 | 37.33 (a*) |
| PB (40 mg/kg) + W-HWE II (25 mg/kg) | 165 ± 14.5 | 133.8 ± 4.171 | 18.91 |
| PB (40 mg/kg)+ W-HWE II (50mg/kg) | 165 ± 92.56 | 121.5 ± 2.217 | 26.36 (a*) |

Values are mean of six independent experiments ± S.D. Comparisons were made between a: PB vs PB+CP, PB+W-HWE II (25 mg/kg) and PB+W-HWE II (50 mg/kg). The symbol (*) represents statistical significance at *p*< 0.05(*).

### TECHNICAL ADVANCEMENTS

The present disclosure described herein above has several technical advantages including, but not limited to, the realization of:
- a process for producing pharmacoactive nutrient from marine algae;
- a pharmacoactive nutrient which enhances the algal biomass production; and
- apharmacoactive nutrient which exhibits a protective effect against central nervous system depression.

The embodiments as described herein above, and various features and advantageous details thereof are explained with reference to the non-limiting embodiments in the description. Descriptions of well-known aspects, components and molecular biology techniques are omitted so as to not unnecessarily obscure the embodiments herein.

The foregoing description of specific embodiments so fully reveal the general nature of the embodiments herein, that others can, by applying current knowledge, readily modify and/or adapt for various applications of such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within scope of the embodiments as described herein. Further, it is to be distinctly understood that the foregoing descriptive matter is to be interpreted merely as illustrative of the disclosure.

Having described and illustrated the principles of the present disclosure with reference to the described embodiments, it will be recognized that the described embodiments can be modified in arrangement and detail without departing from the scope of such principles.

While considerable emphasis has been placed herein on the particular features of this disclosure, it will be appreciated that various modifications can be made, and that many changes can be made in the preferred embodiment without departing from the principles of the disclosure. These and other modifications in the nature of the disclosure or the preferred embodiments will be apparent to those skilled in the art from the disclosure herein, whereby it is to be distinctly understood that the foregoing descriptive matter is to be interpreted merely as illustrative of the disclosure.

## Claims

1. A process for preparing a pharmacoactive nutrient medium from marine algae **characterized in that**, said process comprises the steps of:
- heating an aqueous slurry of live marine algae to a temperature in the range of 90 to 110 °C, for a time period in the range of 25 to 35 minutes to obtain a first mixture;
- cooling said first mixture to 25 to 30 °C to obtain a cooled mixture;
- centrifuging said cooled mixture at a speed of 6000 to 10000 rpm in an environment maintained at 4 °C to obtain a supernatant and a residue; and
- separating said supernatant from said residue and concentrating said residue to 1/4^{th} of its volume to obtain said pharmacoactive nutrient medium.

2. The process as claimed in claim 1, whererin said aqueous slurry is prepared by adding, in a predetermined ratio water and live marine algae in a reaction vessel followed by mixing to form the aqueous slurry.

3. The process as claimed in claim 1, wherein said live marine algae is washed with sterile water at least once prior to adding in said reaction vessel.

4. The process as claimed in claim 1, wherein said live marine algae is selected from the group consisting of *Chlorella vuglaris, Chlorellapyrenoidosa, Chlorella minutissima* and *Chlorella sorokiniana.*

5. The process as claimed in claim 1, wherein said centrifugation is carried out for 20 to 40 minutes.

6. The process as claimed in claim 1, wherein the separation of said supernatant is carried out by decantation or filtration.

7. The process as claimed in claim 1, wherein said pharmacoactive nutrient medium is substantially devoid of heavy metals.

8. The process as claimed in claim 2, wherein said ratio of water and live marine algae is in the range of 100:10 or 100:15.

## Patentansprüche

1. Verfahren zur Zubereitung eines pharmakoaktiven Nährmediums aus Meeresalgen, **dadurch gekennzeichnet, dass** das Verfahren die folgenden Schritte umfasst:
- Erhitzen eines wässrigen Schlamms aus lebenden Meeresalgen auf eine Temperatur im Bereich von 90 bis 110 °C über einen Zeitraum im Bereich von 25 bis 35 Minuten, so dass ein erstes Gemisch erhalten wird;
- Abkühlen des ersten Gemischs auf 25 bis 30 °C, so dass ein abgekühltes Gemisch erhalten wird;
- Zentrifugieren des abgekühlten Gemischs mit einer Geschwindigkeit von 6000 bis 10000 U/min in einer auf 4 °C gehaltenen Umgebung, so dass ein Überstand und ein Rückstand erhalten werden; und
- Abscheiden des Überstands von dem Rückstand und Konzentrieren des Rückstands auf ein Viertel dessen Volumens, so dass das pharmakoaktive Nährmedium erhalten wird.

2. Verfahren nach Anspruch 1, wobei der wässrige Schlamm erzeugt wird durch Zugabe von Wasser und lebenden Meeresalgen in einem vorbestimmten Verhältnis in ein Reaktionsgefäß, gefolgt von Mischen, so dass der wässrige Schlamm gebildet wird.

3. Verfahren nach Anspruch 1, wobei die lebenden Meeresalgen vor der Zugabe in das Reaktionsgefäß mindestens einmal mit sterilem Wasser gewaschen werden.

4. Verfahren nach Anspruch 1, wobei die lebenden Meeresalgen ausgewählt werden aus der Gruppe bestehend aus *Chlorella vulgaris, Chorellapyrenoidosa, Chlorella minutissima* und *Chlorella sorokiniana.*

5. Verfahren nach Anspruch 1, wobei das Zentrifugieren 20 bis 40 Minuten lang durchgeführt wird.

6. Verfahren nach Anspruch 1, wobei das Abscheiden des Überstands durch Dekantieren oder Filtern durchgeführt wird.

7. Verfahren nach Anspruch 1, wobei das pharmakoaktive Nährmedium im Wesentlichen frei ist von Schwermetallen.

8. Verfahren nach Anspruch 2, wobei das Verhältnis von Wasser zu lebenden Meeresalgen im Bereich von 100:10 oder 100:15 liegt.

## Revendications

1. Procédé de préparation d'un milieu nutritif pharmacoactif à partir d'algues marines, **caractérisé en ce que** ledit procédé comprend les étapes suivantes :
chauffage d'une suspension aqueuse d'algues marines vivantes à une température comprise entre 90 et 110 °C, pendant une durée comprise entre 25 et 35 minutes, pour obtenir un premier mélange ;
refroidissement dudit premier mélange entre 25 et 30 °C pour obtenir un mélange refroidi ;
centrifugation dudit mélange refroidi à une vitesse comprise entre 6 000 et 10 000 tr/min dans un environnement maintenu à 4 °C pour obtenir un surnageant et un résidu ; et
séparation dudit surnageant à partir dudit résidu et concentration dudit résidu au 1/4 de son volume pour obtenir ledit milieu nutritif pharmacoactif.

2. Procédé selon la revendication 1, ladite suspension aqueuse étant préparée en ajoutant, dans un rapport prédéfini, de l'eau et des algues marines vivantes dans un récipient de réaction puis en mélangeant pour former la suspension aqueuse.

3. Procédé selon la revendication 1, lesdites algues marines vivantes étant lavées avec de l'eau stérile au moins une fois avant d'être ajoutées dans ledit récipient à réaction.

4. Procédé selon la revendication 1, lesdites algues marines vivantes étant choisies dans le groupe constitué de *Chlorella vulgaris, Chlorellapyrenoidosa, Chlorella minutissima* et *Chlorella sorokiniana.*

5. Procédé selon la revendication 1, ladite centrifugation étant effectuée pendant 20 à 40 minutes.

6. Procédé selon la revendication 1, la séparation dudit surnageant étant effectuée par décantation ou filtration.

7. Procédé selon la revendication 1, ledit milieu nutritif pharmacoactif étant sensiblement dépourvu de métaux lourds.

8. Procédé selon la revendication 2, ledit rapport entre l'eau et les algues marines vivantes étant dans la plage comprise entre 100:10 et 100:15.
